# EUROPEAN PATENT APPLICATION

(11) **EP 3 045 131 A1**
(43) Date of publication of application: **20.07.2016**
(21) Application number: 14843447.5
(22) Date of filing: 14.02.2014
(51) Int. Cl.: A61B 18/00

(54) **TREATMENT TOOL AND SURGICAL SYSTEM**

(30) Priority: 10.09.2013 JP 2013187534
(71) Applicant: Olympus Corporation, Shibuya-ku Tokyo 151-0072 (JP)
(72) Inventor: SUGIYAMA Yuta, Tokyo 151-0072 (JP)
(74) Representative: Winter, Brandl, Fürniss, Hübner, Röss, Kaiser, Polte - Partnerschaft mbB
(86) International application number: PCT/JP2014/053438
(87) International publication number: WO 2015/037253

(57) **Abstract**

A treatment device 20 comprises: a d power reception coil 29 having a solenoid shape that is inductively coupled with a power transmission coil 19 that generates an AC magnetic field, and receives electric power wirelessly; a treatment portion 21 that treats a subject with the electric power received by the power reception coil 29; a horn 22 inserted through an inside of the power reception coil 29; and a magnetic flux concentration member 27 that is made of a soft magnetic material and secured inside of the power reception coil 29.

## Description

### Technical Field

The present invention relates to a treatment device that wirelessly receives electric power through an AC magnetic field, and a surgical system that includes the treatment device.

### Background Art

Endoscopic surgeries have been widely performed for the reason that such endoscopic surgeries are minimally invasive. For example, Japanese Patent Application Laid-Open Publication No. 2009-195676 discloses a surgical system 101 shown in FIG. 1. The surgical system 101 includes a treatment device 120 to be inserted into an abdominal cavity through an insertion hole 110H of a trocar 110 punctured on a body wall of a subject 9.

The treatment device 120 is an ultrasound treatment device and includes a vibration transmission member (horn) 122 that transmits vibration, which is generated by an ultrasound transducer 123 bonded to a back mass 123 A, to a treatment portion 121 located at a distal end of the treatment device. The treatment portion 121 is opened and closed by an operation of a grasping portion 124 grasped by a surgeon, and configured to hold a diseased part to be treated.

The treatment device 120 is connected with a cable 135 for supplying electric power from a power supply unit 130 to the ultrasound transducer. However, the cable 135 becomes a hindrance to a surgeon when performing surgery, which decreases operability of the treatment device.

Japanese Patent Application Laid-Open Publication No. 11-128242 discloses a system that generates an AC magnetic field from a power transmission coil of a trocar and wirelessly supplies electric power to a power reception coil of a treatment device inserted into the trocar.

However, in the surgical system that transmits electric power wirelessly by the AC magnetic field, a conductive body disposed inside the treatment device is induction-heated by an eddy current generated by the AC magnetic field. For example, since the horn of the ultrasound treatment device is made of a high-strength metal, there is a possibility that the horn is heated and operation of the system becomes unstable due to temperature increase in the ultrasound transducer and the treatment portion or decrease in power transmission efficiency.

### Summary of Invention

### Technical Problem

Embodiments of the present invention have an objective to provide a treatment device that achieves a stable operation of wirelessly receiving an AC magnetic field and a surgical system that achieves a stable operation of wirelessly receiving an AC magnetic field.

### Solution to Problem

A treatment device according to an embodiment of the present invention comprises: a power reception coil having a solenoid shape configured to be inductively coupled with a power transmission coil generating an AC magnetic field, and receive an electric power wirelessly; a treatment portion configured to treat a subject with the electric power received by the power reception coil; a conductive body inserted through an inside of the power reception coil; and a magnetic flux concentration member that is made of a soft magnetic material and secured inside of the power reception coil.

Furthermore, a surgical system according to another embodiment of the present invention comprises: a trocar including a power transmission coil having a solenoid shape configured to generate an AC magnetic field, wherein the power transmission coil is wound around an insertion hole of the trocar; a treatment device including: a power reception coil having a solenoid shape configured to be inductively coupled with the power transmission coil and receive an electric power wirelessly when the treatment device is inserted in the insertion port; a treatment portion configured to treat a subject with the electric power received by the power reception coil; a conductive body inserted through an inside of the power reception coil; and a magnetic flux concentration member that is made of a soft magnetic material and secured inside of the power reception coil; and a power supply unit configured to output a driving power to the power transmission coil.

### Advantageous Effects of Invention

Embodiments of the present invention are capable of providing a treatment device that achieves an operation of wirelessly receiving an AC magnetic field to be performed stably and a surgical system that achieves an operation of wirelessly receiving an AC magnetic field to be performed stably.

### Brief Description of the Drawings

FIG. 1 is a pattern diagram of a conventional surgical system.
FIG. 2 is a pattern diagram of a surgical system according to an embodiment.
FIG. 3 is a cross-sectional view of a main part of a treatment device according to a first embodiment.
FIG. 4 is a cross-sectional view of the main part of the treatment device according to the first embodiment, which is taken along IV-IV line in FIG. 3.
FIG. 5 is a transparent perspective view of the main part of the treatment device according to the first embodiment.
FIG. 6A is a perspective view of a magnetic flux concentration member in a modified example of the treatment device according to the first embodiment.
FIG. 6B is a perspective view of a magnetic flux concentration member in another modified example of the treatment device according to the first embodiment.
FIG. 6C is a perspective view of a magnetic flux concentration member in another modified example of the treatment device according to the first embodiment.
FIG. 6D is a perspective view of a magnetic flux concentration member in another modified example of the treatment device according to the first embodiment.
FIG. 7 is a transparent perspective view of a main part of a treatment device according to a second embodiment.
FIG. 8A is a perspective view of a magnetic flux concentration member in a modified example of the treatment device according to the second embodiment.
FIG. 8B is a perspective view of a magnetic flux concentration member in another modified example of the treatment device according to the second embodiment.
FIG. 8C is a perspective view of a magnetic flux concentration member in another modified example of the treatment device according to the second embodiment.
FIG. 8D is a perspective view of a magnetic flux concentration member in another modified example of the treatment device according to the second embodiment.
FIG. 9 is a cross-sectional view of a surgical system according to a third embodiment.

### Mode for Carrying Out the Invention

### <First Embodiment>

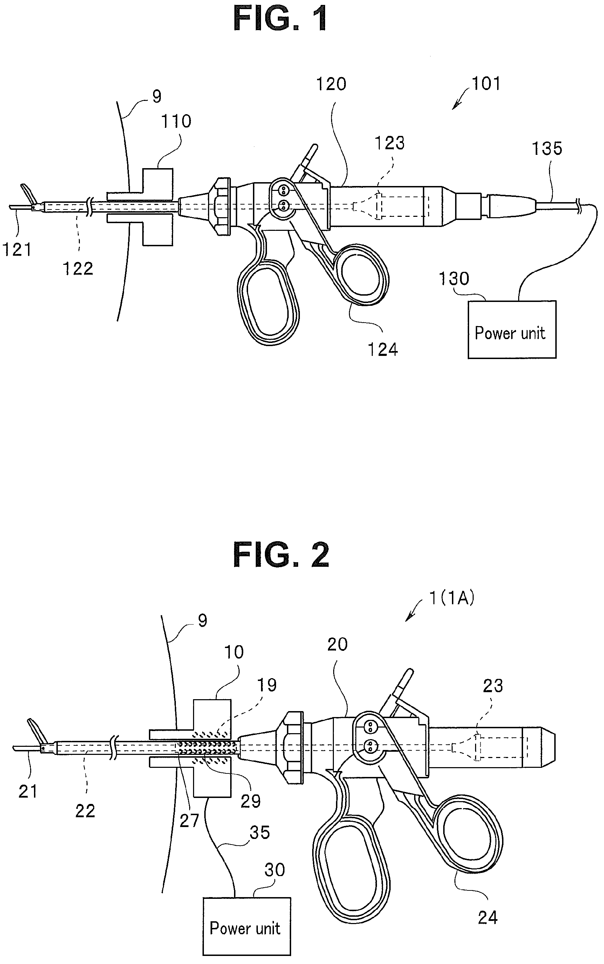

First, with reference to FIGS. 2 to 5, a surgical system 1 and an ultrasound treatment device (hereinafter, also referred to as "treatment device ") 20 according to the first embodiment will be described. As shown in FIG. 2, the surgical system 1 comprises a trocar 10, a power supply unit (power unit) 30, and a treatment device 20. The treatment device 20 for surgery is inserted into a body, for example, the abdominal cavity of a subject 9 through an insertion hole 10H of the trocar 10 punctured on the body wall of the subject 9. Note that, in the surgical system 1, also an endoscope or the like is inserted into the body of the subject 9 through another trocar, but description thereof will be omitted.

The power supply unit 30 outputs 10 to 100w of comparatively large high-frequency driving power, for example. The trocar 10 comprises a power transmission coil 19 which has a solenoid shape and is wound around the insertion hole 10H. When receiving alternating driving power from the power supply unit 30, the power transmission coil 19 generates an AC magnetic field.

The treatment device 20 can be an ultrasound treatment device including: a power reception coil 29; an ultrasound transducer 23; a horn 22, which is a vibration transmission member, made of a conductive body; a treatment portion 21; a magnetic flux concentration member 27; an operation wire 26A; and an electric wire 26B. The treatment device 20 is inserted into the body of the subject 9 through the insertion hole 10H of the trocar 10.

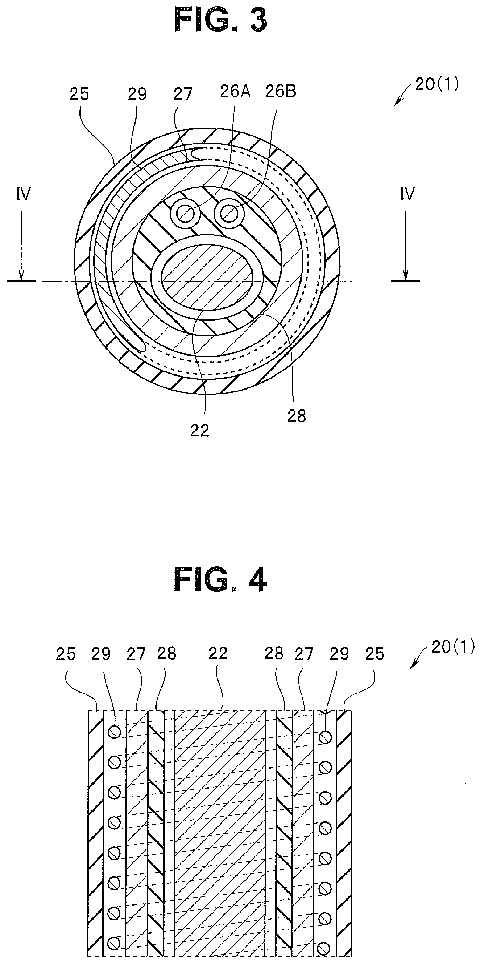

As shown in FIGS. 3 and 4, the horn 22, the operation wire 26A, and the electric wire 26B can be respectively rod-like or wire-like constituent elements, which are inserted through respective lumens of a multi-lumen tube 28. In addition, the outer circumference of the power reception coil 29 is covered with an outer packaging tube 25 made of a resin with high biocompatibility.

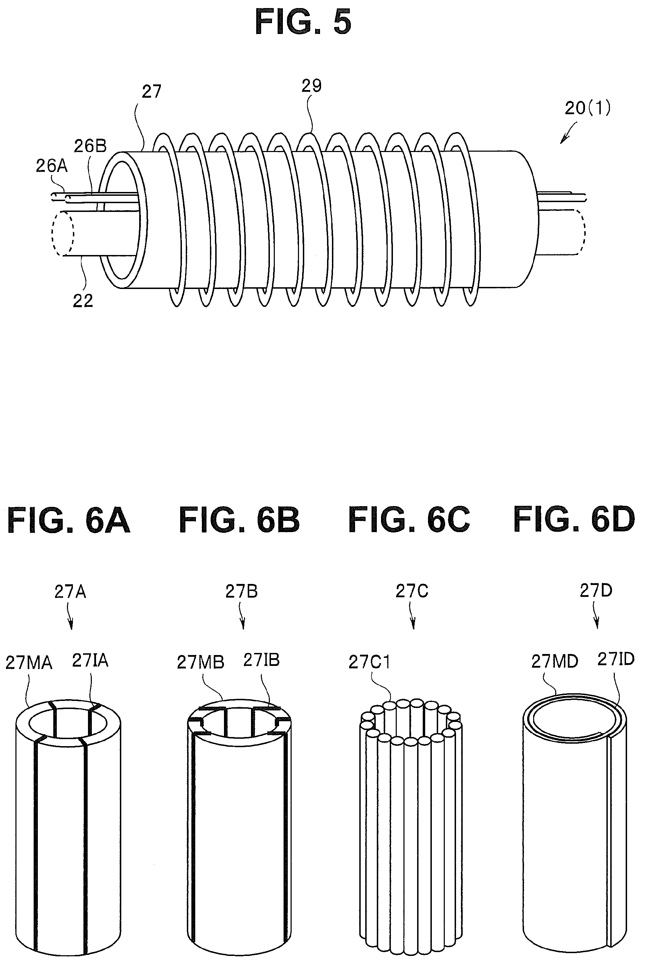

As shown in FIG. 5 and the like, the power reception coil 29 wound around the outer circumference of the multi-lumen tube 28 has a solenoid shape and the longitudinal axis direction of the power reception coil is the longitudinal direction of the treatment device 20. When the treatment device 20 is inserted into the insertion hole 10H, the power reception coil 29 is brought into a state being inserted concentrically in the power transmission coil 19, and the power reception coil 29 is inductively coupled with the power transmission coil 19, to wirelessly receive electric power.

The power transmission coil 19 configures a power-transmission side LC series resonance circuit including a power transmission circuit (not shown) having a power transmission capacitor, and generates an AC magnetic field with a predetermined resonant frequency FR1. In addition, the power reception coil 29 configures a power-reception side LC series resonance circuit including a power reception circuit (not shown) having a power reception capacitor, and effectively receives an AC magnetic field with a predetermined resonant frequency FR2.

The resonant frequency FR1 of the power-transmission side LC series resonance circuit and the resonant frequency FR2 of the power reception side LC series resonance circuit are substantially same, and wireless power transmission and reception are effectively performed by magnetic field resonance phenomenon in the surgical system 1. Note that the resonant frequencies FR1 and FR2 are appropriately selected within a range of 10 kHz to 20 MHz, for example.

A grasping portion 24 of the treatment device 20 is grasped and operated by a surgeon. When the driving power received by the power reception coil 29 is applied to the ultrasound transducer 23 configured by a multi-layer type piezoelectric element, the ultrasound transducer 23 ultrasonically vibrates. The proximal end portion of the ultrasound transducer 23 is mechanically coupled with a back mass 23 A made of metal.

The horn 22 has a rod-like shape, and transmits the vibration of the ultrasound transducer 23 to the treatment portion 21. That is, the horn 22 has the proximal end portion mechanically coupled with the ultrasound transducer 23 and the distal end portion mechanically coupled with the treatment portion 21. The horn 22 is made of a high strength metal, i.e., titanium alloy such as 64 titanium alloy, or pure titanium, in order to effectively transmit the vibration.

The treatment portion 21 comprises a vibration portion that ultrasonically vibrates and a holding portion to be paired with the vibration portion. When the operation of the grasping portion 24 is transmitted to the treatment portion 21 through the operation wire 26A for opening and closing the distal end treatment portion, a diseased part that is a target to be treated is held between the vibration portion and the holding portion. When the vibration portion vibrates with the diseased part being held, ultrasound vibration is applied to the diseased part and the diseased part is treated.

In the surgical system 1, the cable 35 extended from the power supply unit 30 is connected to the trocar 10. The treatment device 20 is configured such that the ultrasound transducer 23 is driven with the electric power wirelessly received by the power reception coil 29, which eliminates a need for providing a power-supply cable in the treatment device 20 and provides excellent operability.

The hollow cylindrical-shaped magnetic flux concentration member 27 secured inside the power reception coil 29 is made of a soft magnetic material having high magnetic permeability µ, for example, soft ferrite, permalloy, amorphous alloy, or the like. The soft magnetic material is made of a material, the magnetic permeability µ of which is 100 or more, preferably, 1000 or more at the frequency of the driving signal, that is, the resonant frequency FR1. If the magnetic permeability µ is equal to or larger than a numerical value in the above-described range, the magnetic flux concentration effect can be sufficiently obtained in the cross-sectional area which allows the magnetic flux concentration member 27 to be secured inside the treatment device 20. Note that the upper limit of the magnetic permeability µ is not specifically limited, but is technically 100000, for example.

In the surgical system 1, even if the treatment device 20 is inserted in the trocar 10 and the power reception coil 29 is brought into a state of receiving the AC magnetic field generated by the power transmission coil 19 (inductively coupled state), the AC magnetic field generated by the power transmission coil 19 concentrates on the magnetic flux concentration member 27 in the power reception coil 29. As a result, a strong magnetic field is not applied to the horn 22, and the like.

The horn 22, the operation wire 26A, and the electric wire 26B are configured by conductive bodies, and inserted through inside the power reception coil 29. However, the horn 22 and the like are not induction-heated by the AC magnetic field, which does not cause temperature increase in the ultrasound transducer 23 and the treatment portion 21 by the generation of eddy current. In addition, the power transmission efficiency is not decreased in the surgical system 1. Therefore, the operations of the treatment device 20 and the surgical system 1 are stable.

Note that the magnetic flux concentration member 27 shown in FIG. 5 and the like is inserted through inside the power reception coil 29. Even if the length of the magnetic flux concentration member 27 is shorter than the length of the power reception coil 29, a heat-generation prevention effect can be obtained.

In addition, the ultrasound treatment device has been described above as the treatment device 20. However, even if other various kinds of treatment devices configured to have a conductive body in the power reception coil 29, such as an electrocautery scalpel, or a high-frequency forceps, are used, for example, the same effects can be obtained.

Another conductive member other than the horn 22, the operation wire 26A, and the electric wire 26B may be secured inside the power reception coil 29, or another conductive member which is not inserted through the power reception coil 29 may be secured.

### <Modified Example of First Embodiment>

Next, modified examples 1 to 4 of the treatment device according to the first embodiment will be described with reference to FIGS. 6A to 6D. The treatment devices and the surgical systems in the modified examples 1 to 4 are different from the treatment device 20 and the surgical system 1 in the first embodiment only in the configuration of the magnetic flux concentration member, and other configurations are the same as those of the first embodiment. Therefore, only the magnetic flux concentration member will be described.

The magnetic flux concentration efficiency, that is, the magnetic permeability µ of the magnetic flux concentration member decreases as the frequency of the AC magnetic field increases. In the surgical system, since electric power is wirelessly transmitted, the frequency of the AC magnetic field generated by the power transmission coil 19 is relatively high, for example, 10kHz to 20 MHz. As a result, the surgical system is likely to be influenced by loss and decrease of electric power caused especially by the generation of eddy current.

If the specific resistance of the soft magnetic material of the magnetic flux concentration member is set to be high, the power loss and decrease can be suppressed. However, in view of the cost and the like, the modified examples 1 to 4 to be described below are more preferable.

That is, each of the magnetic flux concentration members according to the modified examples 1 to 4 is configured such that the conductive soft magnetic material is divided by insulation layers made of resin and the like. Therefore, the treatment devices and the surgical systems according to the respective modified examples 1 to 4 have the effects of the treatment device 20 and the surgical system 1, and in addition, the operations are similarly stable even if the volumes of the magnetic flux concentration members are small.

A magnetic flux concentration member 27A in the modified example 1 shown in FIG. 6A is configured by a plurality of members 27MA formed by dividing the magnetic flux concentration member 27A into four parts in the longitudinal axis direction along the circumference, and insulating materials 27IA that insulate between the respective members 27MA. In other words, the magnetic flux concentration member 27A has dividing surfaces (cut surfaces) on the surfaces parallel to the longitudinal axis direction and the dividing surfaces are insulated. Note that, if the magnetic flux concentration member 27A has the cut surface at one position at least, a predetermined effect can be obtained. There is no specific upper limit on the number of divisions, but in the case where the number of divisions is ten or more, for example, there is no noticeable difference in the effect.

A magnetic flux concentration member 27B according to the modified example 2 shown in FIG. 6B is configured by a plurality of members 27MB formed by dividing the magnetic flux concentration member 27B into four parts in the longitudinal axis direction so as to be parallel to the circumference, and insulating materials 27IB that insulate between the respective members 27MB

A magnetic flux concentration member 27C according to the modified example 3 shown in FIG. 6C is configured by a plurality of rod-like (columnar) members 27C, the circumferences of which are respectively covered with insulating materials. The magnetic flux concentration member 27C may be rectangular cylinders, or the like.

A magnetic flux concentration member 27D according to the modified example 4 shown in FIG. 6D is configured by a thin ribbon 27MD made of a soft magnetic material, which is wound with an insulation layer 27ID interposed. In other words, the magnetic flux concentration member 27D is configured such that the cross section perpendicular to the longitudinal axis has a spiral shape, and the contact parts of the layered thin ribbon 27MD are insulated. The thin ribbon 27MD can be made of an amorphous thin ribbon manufactured by high-speed quenching method, for example.

The magnetic permeability µ of the thin ribbon 27MD is not likely to reduce due to the skin effect of the thin ribbon. Therefore, the thin ribbon 27MD is capable of effectively concentrating the magnetic flux.

### <Second Embodiment>

Next, description will be made on a surgical system 1A and a treatment device 20A according to the second embodiment. The surgical system 1A and the like are similar to the surgical system 1 and the like. The same constituent elements as those in the surgical system 1 and the like are attached with the same reference numerals and descriptions thereof will be omitted.

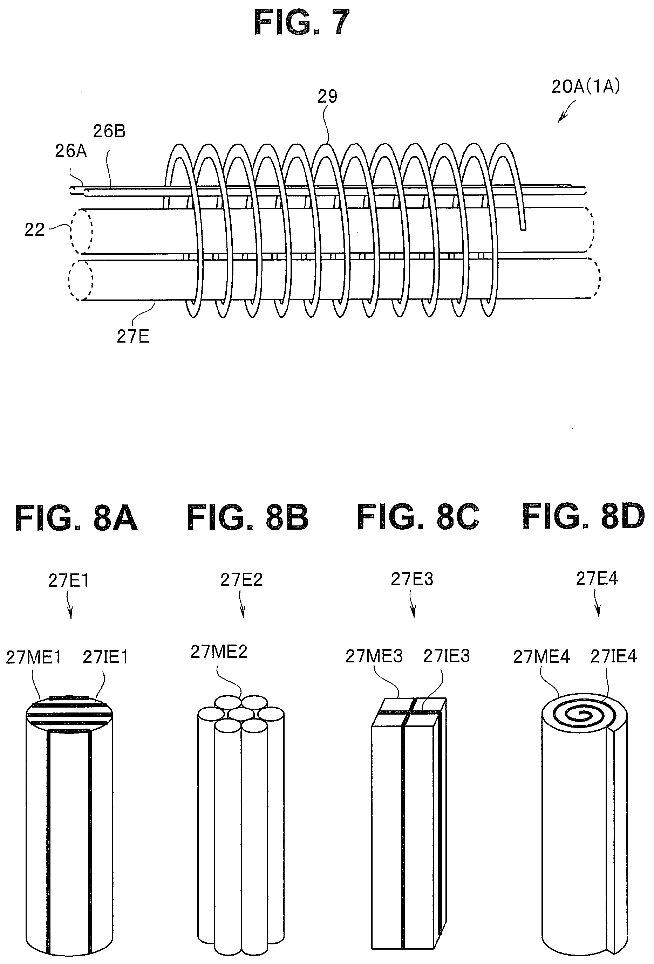

As shown in FIG. 7, the treatment device 20A of the surgical system 1A comprises a magnetic flux concentration member 27E which has a rod-like shape. The magnetic flux concentration member 27E is made of a material similar to that of the magnetic flux concentration member 27. Therefore, even if the magnetic flux concentration member 27E has the rod-like shape, the similar effects as those of the magnetic flux concentration member 27 can be obtained. In addition, since the rod-like shaped magnetic flux concentration member 27E can be manufactured by extruding molding, for example, the magnetic flux concentration member 27E is easier to be manufactured and has a higher degree of disposing freedom than the hollow cylindrical-shaped magnetic flux concentration member 27.

Note that the cross-sectional shape of the magnetic flux concentration member 27E may be rectangular, polygonal, or the like. For example, the magnetic flux concentration member 27E may be secured in a lumen, which has a circular cross section, of a multi-lumen tube (see FIG. 4, etc.).

Note that it is preferable that the central axis of the magnetic flux concentration member 27E is eccentric from the central axis of the power reception coil 29, in order to increase the degree of disposing freedom for other members. In addition, a thick constituent element can be inserted in the power reception coil 29.

Furthermore, the treatment device 20A comprises the one magnetic flux concentration member 27E. However, a plurality of rod-like shaped magnetic flux concentration members may be secured in the power reception coil 29.

### <Modified Example of Second Embodiment>

Next, description will be made on modified examples 1 to 4 of the treatment device in the second embodiment, with reference to FIGS. 8A to 8D. The treatment devices and the surgical systems according to the modified examples 1 to 4 are different from the treatment device 20A and the surgical system 1 A according to the second embodiment only in the configuration of the magnetic flux concentration member, and other configurations are the same as those of the treatment device 20A and the surgical system 1A. Therefore, description will be made only on the magnetic flux concentration member.

The magnetic flux concentration member according to each of the modified examples 1 to 4 comprises a conductive soft magnetic material divided by insulation layers, similarly as in the magnetic flux concentration member of the treatment device 20 according to the first embodiment. Therefore, the treatment devices and the surgical systems according to the modified examples 1 to 4 have the effects of the treatment device 20A and the surgical system 1A, and operations of the treatment devices and the surgical systems in the respective modified examples are similarly stable even if the volumes of the magnetic flux concentration members are small.

A magnetic flux concentration member 27E1 according to the modified example 1 shown in FIG. 8A is configured by a plurality of members 27ME1 formed by dividing the magnetic flux concentration member 27E1 into four parts in the longitudinal axis direction so as to be parallel to the circumference, and insulating materials 27IE1 that insulate between the respective members 27ME1.

A magnetic flux concentration member 27E2 according to the modified example 2 shown in FIG. 8B is configured by a plurality of columnar members 27ME2, the outer circumferences of which are respectively covered with insulating materials. Note that columnar soft magnetic materials, which are not covered with the insulating materials, may be secured in different lumens of a multi-lumen tube.

A magnetic flux concentration member 27E3 according to the modified example 3 shown in FIG. 8C is configured by a plurality of rectangular column-shaped members 27ME3 that are respectively insulated by insulating materials 27IE3. The rectangular column-shaped members 27ME3 can be disposed more densely than the columnar members 27ME2.

A magnetic flux concentration member 27E4 according to the modified example 4 shown in FIG. 8D is configured by a thin ribbon 27ME4 made of a soft magnetic material, which is wound with an insulation layer 27IE4 interposed.

### <Third Embodiment>

Next, an endoscope system 1B and a treatment device 20B according to the third embodiment will be described. The surgical system 1B and the like are similar to the surgical system 1 and the like. The same constituent elements as those in the surgical system 1 and the like are attached with the same reference numerals and descriptions thereof will be omitted.

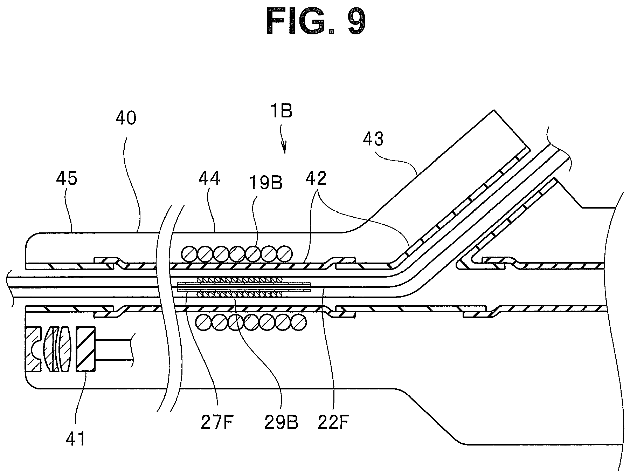

As shown in FIG. 9, the surgical system 1B comprises an endoscope 40 and a treatment device 20B, which are configured to be inserted into a body of a subject. The endoscope 40 comprises: an elongated insertion portion 44 that comprises, at a distal end portion 45, an image pickup device 41; a grasping portion 43 secured on the proximal end portion side of the insertion portion 44; and a universal cord (not shown) extended from the grasping portion 43 to be connected to a processor. A channel 42 is inserted through inside of the insertion portion 44 from the grasping portion 43 to the distal end portion 45. The treatment device 20B is inserted from the grasping portion 43 into the channel 42.

A power transmission coil 19B having a solenoid shape is wound around the channel of the endoscope 40. The power transmission coil 19B is connected to a power supply unit (not shown).

In the surgical system 1B, the treatment device 20B comprises a power reception coil 29B to be arranged concentrically with the power transmission coil 19B and inductively coupled with the power transmission coil 19B when the treatment device 20B is inserted into the channel 42. Treatment is performed by the treatment portion (not shown) located at the distal end of the treatment device, with electric power wirelessly received by the power reception coil 29B. In addition, inside the power reception coil 29B, an electric wire 22F having a core made of copper, and the like are inserted.

The treatment device 20B comprises, inside the power reception coil 29B, a magnetic flux concentration member 27F made of a soft magnetic material, which is similar to the magnetic flux concentration members 27 to 27E. Therefore, the electric wire 22F made of a conductive body is not likely to be induction-heated to generate heat. Furthermore, there is no possibility that the power transmission efficiency is decreased in the endoscope system 1B and the treatment device 20B. This leads to stable operations of the surgical system 1B and the treatment device 20B.

Note that, in order to ensure the flexibility of the insertion portion 44, it is preferable for the magnetic flux concentration member 27F to have flexibility. For example, it is preferable to use a composite magnetic body configured by soft magnetic material particles being dispersed in a flexible resin or a magnetic body made of a thin line (wire), as the magnetic flux concentration member 27F.

The present invention is not limited to the above-described embodiments, and various changes and modifications are possible without changing the gist of the present invention.

The present application is filed claiming the priority of Japanese Patent Application No. 2013-187534 filed in Japan on September 10, 2013, and the above described disclosure is incorporated by reference in the present description, claims, and drawings.

## Claims

1. A treatment device comprising:
a power reception coil having a solenoid shape configured to be inductively coupled with a power transmission coil generating an AC magnetic field, and receives an electric power wirelessly;
a treatment portion configured to treat a subject with the electric power received by the power reception coil;
a conductive body inserted through an inside of the power reception coil;
and
a magnetic flux concentration member that is made of a soft magnetic material and secured inside of the power reception coil.

2. The treatment device according to claim 1,
wherein the magnetic flux concentration member has a hollow cylindrical shape through which the conductive body is inserted.

3. The treatment device according to claim 1,
wherein the magnetic flux concentration member has a rod-like shape.

4. The treatment device according to claim 2 or 3,
wherein the magnetic flux concentration member comprises a plurality of members formed by dividing the magnetic flux concentration member in a longitudinal axis direction,
wherein the plurality of members are insulated from each other.

5. The treatment device according to claim 2,
wherein the magnetic flux concentration member is formed by a thin ribbon being wound with an insulation layer interposed,
wherein the thin ribbon is made of a soft magnetic material.

6. The treatment device according to any one of claims 1 to 5,
wherein the magnetic flux concentration member is inserted through the power reception coil.

7. The treatment device according to claim 6,
wherein the power transmission coil is a coil having a solenoid shape wound around an insertion hole of a trocar.

8. The treatment device according to any one of claims 1 to 7, further comprising,
a transducer configured to generate an ultrasonic vibration with the electric power received by the power reception coil,
wherein the conductive body is a vibration transmission member made of rod-like shaped metal configured to transmit the ultrasonic vibration,
wherein the conductive body is mechanically coupled with a proximal end portion of the vibration transmission member, and
wherein the treatment portion is mechanically coupled with a distal end portion of the vibration transmission member and configured to apply the ultrasonic vibration to a target of the subject for the treatment.

9. The treatment device according to any one of claims 1 to 6,
wherein the power transmission coil is a coil having a solenoid shape wound around a channel inserted through an insertion portion of an endoscope, and the treatment device is inserted in the channel.

10. A surgical system comprising:
a trocar comprising a power transmission coil having a solenoid shape configured to generate an AC magnetic field, wherein the power transmission coil is wound around an insertion hole of the trocar;
a treatment device comprising:
a power reception coil having a solenoid shape configured to be inductively coupled with the power transmission coil and receive an electric power wirelessly when the treatment device is inserted in the insertion port;
a treatment portion configured to treat a subject with the electric power received by the power reception coil;
a conductive body inserted through an inside of the power reception coil; and
a magnetic flux concentration member that is made of a soft magnetic material and secured inside of the power reception coil; and
a power supply unit configured to output a driving power to the power transmission coil.
